# EUROPÄISCHE PATENTANMELDUNG

(11) **EP 2 165 769 A2**
(43) Veröffentlichungstag der Anmeldung: **24.03.2010**
(21) Anmeldenummer: 09011454.7
(22) Anmeldetag: 08.09.2009
(51) Int. Cl.: B05B 1/30, B05B 9/08, B05B 17/06, B05C 5/02, B05C 11/10

(54) **Austragvorrichtung**

(30) Priorität: 19.09.2008 US 284244
(71) Anmelder: Ing. Erich Pfeiffer GmbH, 78315 Radolfzell (DE)
(72) Erfinder: Cater, Miro, Daytona Beach, Florida 32124 (US)
(74) Vertreter: Patentanwälte Ruff, Wilhelm, Beier, Dauster & Partner

(57) **Zusammenfassung**

Die Erfindung betrifft eine Austragvorrichtung und die Verwendung einer Austragsvorrichtung für ein flüssiges pharmazeutisches Medium mit einem Reservoir zur Aufbewahrung des Mediums, einer Druckbeaufschlagungsvorrichtung (10) zur Förderung des Mediums und mindestens einer Auslassöffnung (80) zur Abgabe des Mediums an eine Umgebung, wobei die Druckbeaufschlagungsvorrichtung (10) eine Druckkammer (16) aufweist, deren Inhalt mittels eines translativ beweglichen Kolbens (14) druckbeaufschlagbar ist, wobei
eine Piezoaktuatoreinrichtung mit einem Piezoaktuator, der mit dem Kolben wirkgekoppelt ist, oder eine Spulenaktuatoreinrichtung (30) mit einer Aktuatorspule (30) und einem Aktuatorspulenkern (32) der durch Bestromung der Aktuatorspule (30) gegenüber der Aktuatorspule (30) kraftbeaufschlagbar ist, vorgesehen ist, wobei entweder die Aktuatorspule (30) oder der Aktuatorspulenkern (32) ortsfest zum Kolben ausgebildet ist.

## Beschreibung

Die Erfindung betrifft eine Austragvorrichtung für ein flüssiges pharmazeutisches Medium mit einem Reservoir zur Aufbewahrung des Mediums, einer Druckerzeugungsvorrichtung zur Förderung des Mediums und mindestens einer Auslassöffnung zur Abgabe des Mediums an eine Umgebung, wobei die Druckbeaufschlagungsvorrichtung eine Druckkammer aufweist, deren Inhalt mittels eines translativ beweglichen Kolbens druckbeaufschlagbar ist.

Gattungsgemäße Austragvorrichtungen sind in vielfältiger Art aus dem Stand der Technik bekannt. Sie dienen der Abgabe pharmazeutischer Medien, wobei unter pharmazeutischen Medien im Zusammenhang mit dieser Erfindung Substanzen zu verstehen sind, die zu medizinischen Zwecken in oder auf dem Körper eines Patienten ausgebracht werden.

Dabei kann der Austrag abhängig von der Austragvorrichtung in Tröpfchenform, in Form eines Strahls, eines Nebels oder ähnlich erfolgen.

Das Reservoir dient bei gattungsgemäßen Austragvorrichtungen der Speicherung des Mediums vor dessen Austrag. Zum Austrag wird ein Teil des Mediums, welches zuvor aus dem Reservoir in die Druckkammer gefördert wurde, druckbeaufschlagt, um es zur Auslassöffnung zu fördern.

Die Betätigung der vorzugsweise als Pumpvorrichtung ausgebildeten Druckerzeugungsvorrichtung erfolgt bei den meisten aus dem Stand der Technik bekannten Austragvorrichtungen manuell, also durch eine Druckbeaufschlagung, bei der die erforderliche Energie durch den Bediener in das System eingebracht wird. Dies bringt eine Vielzahl von Nachteilen mit sich, wobei insbesondere die für ältere Menschen mitunter schwierige Bedienung und die Möglichkeit der Fehlbedienung zu nennen sind.

### Aufgabe und Lösung

Aufgabe der Erfindung ist es daher, eine gattungsgemäße Austragvorrichtung dahingehend weiterzubilden, dass die Nachteile des Standes der Technik vermindert oder vermieden werden.

Erfindungsgemäß wird dies durch eine gattungsgemäße Austragvorrichtung erreicht, die über eine Piezoaktuatoreinrichtung oder eine Spulenaktuatoreinrichtung verfügt, wobei die Piezoaktuatoreinrichtung einen Piezoaktuator aufweist, der mit dem Kolben wirkgekoppelt ist, oder wobei die Spulenaktuatoreinrichtung eine Aktuatorspule und einen Aktuatorspulenkern aufweist, der durch Bestromung der Aktuatorspule gegenüber der Aktuatorspule kraftbeaufschlagbar ist, wobei entweder die Aktuatorspule oder der Aktuatorspulenkern ortsfest zum Kolben ausgebildet ist.

Im Sinne dieser Erfindung wird als Kolben ein gegenüber einem Gehäuse der Austragvorrichtung translativ beweglicher Abschnitt verstanden, dessen Lage das Volumen der Druckkammer bestimmt. Ein Kolben im Sinne dieser Erfindung kann auch die bewegliche Stirnseite eines Pumpenbalgs einer Balgpumpe sein.

Bei der Ausführungsform mit Piezoaktuatoreinrichtung ist vorgesehen, dass der Piezoaktuator, der in zumindest einer Dimension durch Bestromung seine Ausdehnung ändert, sich mit einer Seite an einer zu einem Gehäuse der Austragvorrichtung ortsfesten Fläche abstützt, während die gegenüberliegende Seite durch die Längenausdehnung des Piezoaktuators beweglich ist und mit dem Kolben mittelbar oder unmittelbar derart verbunden ist, dass die Bewegung dieser gegenüberliegenden Seite eine Bewegung des Kolbens bewirkt. Der Piezoaktuator ist vorzugsweise als Piezo-Stack ausgebildet, um eine vergleichsweise große Verlagerung der beweglichen Seite zu erzielen. Piezoaktuatoren eignen sich aufgrund der hohen erzielbaren Kräfte sowie der genauen Dosierbarkeit der Kräfte besonders gut zur Kraftbeaufschlagung des Kolbens und erlauben die Herstellung eines definierten Drucks in der Druckkammer.

Besonders von Vorteil ist es, wenn die Piezoaktuatoreinrichtung über einen Umsetzer mit dem Kolben wirkgekoppelt ist, wobei der Umsetzer dafür ausgebildet ist, den Kolben in Folge einer Verformung des Piezoaktuators um eine Weglänge L1 um eine Weglänge L2 zu verlagern, die größer als die Weglänge L1 ist. Der Umsetzer ist demnach dafür ausgebildet, die vergleichsweise geringe Verformungsstrecke des Piezoaktuators unter entsprechender Verringerung der Kräfte zu verlängern. Dies ermöglicht es, einen großen Hub des Kolbens zu erzielen. Der Umsetzer kann als mechanisch wirkendes Getriebe ausgebildet sein, welches beispielsweise über einen ortsfest gelagerten Hebel eine Umsetzung erzielt. Ebenfalls möglich ist es, einen hydraulischen Umsetzer zu verwenden, der so geartet ist, dass durch den Piezoaktuator unmittelbar ein Hilfskolben mit einer großen Kolbenfläche bewegt wird, der ein Hilfsfluid verdrängt, welches seinerseits zur Bewegung eines zweiten Hilfskolbens mit einer vergleichsweise kleinen Kolbenfläche genutzt wird. Mit diesem zweiten Hilfskolben kann der Hauptkolben, der die Druckkammer begrenzt, unmittelbar verbunden sein.

Bei der Gestaltung mit einer Spulenaktuatoreinrichtung wird die Tatsache genutzt, dass eine bestromte Spule ein Magnetfeld ausbildet, in dem der Aktuatorenspulenkern durch die Magnetkräfte kraftbeaufschlagt wird. Um diesen Effekt zu vergrößern, ist der Aktuatorenspulenkern vorzugsweise als Permanentmagnet ausgebildet. Die auf den Aktuatorenspulenkern wirkende Kraft hängt von der Stromstärke des Stroms ab, der in der Aktuatorenspule fließt.

Die beschriebene Kraft kann unmittelbar verwendet werden, um den Kolben zu verlagern bzw. mit einer Kraft zu beaufschlagen. Hierzu ist es möglich, die Aktuatorspule ortsfest zum Pumpengehäuse vorzusehen und den Aktuatorspulenkern ortsfest zum Kolben anzuordnen. Jede Bewegung und Kraftbeaufschlagung des Aktuatorspulenkerns äußert sich somit in einer entsprechenden Bewegung bzw. Kraftbeaufschlagung des Kolbens. Diese Bauweise ist besonders einfach, da nur ortsfeste Bestandteile mit einer Stromversorgung ausgestattet sein müssen.

Alternativ ist es jedoch auch möglich, den Aktuatorspulenkern ortsfest zum Pumpengehäuse vorzusehen und die Aktuatorspule am Kolben anzuordnen. Diese zweite Ausgestaltung, die bezüglich ihrer Bauweise insoweit in etwa einem Lautsprecher entspricht, weist den Vorteil auf, dass die gegenüber dem Aktuatorspulenkern vergleichsweise leichte Aktuatorspule bewegt wird, so dass der Energiebedarf geringer ist.

Bei einer Weiterbildung der Erfindung ist eine Messeinrichtung vorgesehen, mittels derer die Auslenkung des Kolbens zu einem Pumpengehäuse erfassbar ist.

Die Messeinrichtung kann im einfachsten Fall derart ausgebildet sein, dass sie lediglich erfasst, ob eine Kolbenbewegung vorliegt. Sie kann jedoch auch derart ausgebildet sein, dass sie die Lage des Kolbens präzise erfassen kann. Die Messeinrichtung gestattet es unter anderem, vor in Betriebnahme oder im Betrieb zu überprüfen, ob der Kolben sich in Reaktion auf eine Bestromung des Piezoaktuators oder der Aktuatorspule bewegt. Bei komplexeren Ausgestaltungen erlaubt es die Messeinrichtung, zusätzlich zu erfassen, welche weiteren Kräfte, wie beispielsweise Reibungskräfte oder Federkräfte, auf den Kolben wirken. Eine derartige Analyse der Kenndaten der spezifischen Austragvorrichtung erlaubt es, im Betrieb einen genau definierten Druck in der Druckkammer zu erzeugen, was in Abhängigkeit des Anwendungsgebietes vielfältige Vorteile bietet.

Besonders von Vorteil ist eine Austragvorrichtung, bei der die Messeinrichtung eine Messspule aufweist, die zur Erfassung der Lage eines Messspulenkerns relativ zur Messspule ausgebildet ist. Dabei wird der Effekt genutzt, dass eine Bewegung des permanentmagnetischen Messspulenkerns zur Induzierung einer Spannung in der Messspule führt. Diese Spannung ist umso größer, je größer die Geschwindigkeit des Messspulenkerns relativ zur Messspule ist. Damit kann sowohl erfasst werden, ob sich der Messspulenkern bewegt, als auch, wie schnell er sich bewegt. Schon die Erfassung der Tatsache, dass der Spulenkern sich bewegt, erlaubt es, die zwischen dem Kolben und dem Pumpenzylinder wirkenden Reibungskräfte zu erfassen, indem der Kolben zwischen einer ersten und einer zweiten Endlage bewegt wird und dabei die Zeit der Bewegung erfasst wird. Diese ist umso größer, je größer die Reibungskräfte sind.

Der in der Druckkammer erzeugte Druck kann unmittelbar zum Austrag des Mediums durch die Auslassöffnung genutzt werden. Hierfür besteht eine direkte Verbindung zwischen der Druckkammer und der Auslassöffnung, wobei vorzugsweise ein Auslassventil vorgesehen ist, welches erst bei Erreichen eines spezifischen Mindestdrucks öffnet. Alternativ dazu ist es jedoch auch möglich, dass mittels der Pumpvorrichtung geförderte Medium zunächst in eine andere Kammer zu fördern, aus der es mittels eines speziellen Austragmechanismus ausgetragen wird.

So ist es bevorzugt, dass eine mit der Druckkammer verbundene Auslasskammer vorgesehen ist, wobei die Auslasskammer durch eine Mehrzahl von Auslassöffnungen mit der Umgebung verbunden ist und wobei Auslasskammer durch einen Wandungsabschnitt begrenzt wird, der durch einen Vibrationsaktuator in einen Vibrationszustand versetzbar ist.

Bei einer solchen Gestaltung wird der eigentliche Austragvorgang durch den vibrierenden Wandungsabschnitt bewirkt, der zu einem hochfrequent pulsierenden Volumen der Auslasskammer führt. Diese Volumenänderung führt dazu, dass das in der Druckkammer vorhandene Medium durch die Auslassöffnung in Form eines Nebels mit winzigen Tröpfchen hindurch austritt. Der durch die Pumpeinrichtung erzeugte Druck dient dabei lediglich der Versorgung der Auslasskammer mit Medium. Insbesondere bei einer solchen Gestaltung ist die Verwendung einer erfindungsgemäßen Piezoaktuatoreinrichtung oder einer erfindungsgemäßen Spulenaktuatoreinrichtung von Vorteil, da diese die Zurverfügungstellung eines genau dosierten und nur geringen Drucks ermöglichen, der so bemessen ist, dass die Versorgung der Auslasskammer mit Medium gewährleistet ist, ohne dass allein schon aufgrund des von der Pumpvorrichtung erzeugten Drucks ein Austritt durch die Auslassöffnungen erfolgt.

Zur Steuerung der Pumpvorrichtung ist bei einer Weiterbildung ein Steuergerät vorgesehen, welches mittels der Piezoaktuatoreinrichtung oder der Spulenaktuatoreinrichtung eine Kraftbeaufschlagung des Kolbens steuert. Die Steuerung erfolgt im Falle einer Piezoaktuatoreinrichtung dadurch, dass das Steuergerät eine definierte Spannung zur Verfügung stellt. Bei einer Spulenaktuatoreinrichtung wird durch das Steuergerät eine definierte Stromstärke zur Verfügung gestellt. Die Spannung bzw. die Stromstärke beeinflussen unmittelbar die Kraftbeaufschlagung des Kolbens. Unter Einbeziehung der Kolbenfläche ist hierdurch ein definierter Druck in der Druckkammer erzeugbar. Um diesen definierten Druck unabhängig von anderen Einflussfaktoren wie Reibungskräften zwischen Kolben und Pumpenzylinderwandung zu erzielen, können zuvor von der Messeinrichtung erfasste Werte einbezogen werden, beispielsweise die erforderliche Zeit, die im noch leeren Zustand der Druckkammer gebraucht wird, um den Kolben von einer Endlage in die andere Endlage zu überführen. Zu diesem Zweck ist das Steuergerät vorzugsweise zusätzlich dafür ausgebildet ist, auf den Kolben wirkende Reibungs- und/oder Federkräfte durch eine Auswertung der von der Messeinrichtung während einer Verlagerung des Kolbens ermittelten Werte zu erfassen.

Weiterhin kann das Steuergerät dafür ausgebildet sein, bei der Inbetriebnahme der Austragvorrichtung mittels einer Messung der Verlagerung des Kolbens zu ermitteln, ob in einem Strömungspfad zwischen der Pumpvorrichtung und der Austragöffnung noch Luft eingeschlossen ist. Das Steuergerät kann darüber hinaus für mannigfaltige andere Zwecke verwendet werden, beispielsweise um die Zahl der Austragvorgänge zu zählen oder einen Austragvorgang zu verhindern, sofern seit einem vorangegangenen Austragvorgang nicht eine bestimmte Zeitspanne verstrichen ist.

### Kurzbeschreibung der Zeichnungen

Weitere Aspekte und Merkmale der Erfindung ergeben sich außer aus den Ansprüchen auch aus der nachfolgenden Beschreibung dreier bevorzugter Ausführungsbeispiele der Erfindung, die in den Zeichnungen dargestellt sind und nachfolgend beschrieben werden. In den Zeichnungen zeigen:
- Fig. 1: eine erste Ausführungsform einer erfindungsgemäßen Austragvorrichtung mit einer Spulenaktuatoreinrichtung und einem druckabhängig öffnenden Auslassventil,
- Fig. 2: eine zweite Ausführungsform einer erfindungsgemäßen Austragvorrichtung mit einer Spulenaktuatoreinrichtung und einem Nebelerzeuger und
- Fig. 3: eine dritte Ausführungsform einer erfindungsgemäßen Austragvorrichtung mit einer Piezoaktuatoreinrichtung und einem druckabhängig öffnenden Auslassventil.

### Detaillierte Beschreibung der Ausführungsbeispiele

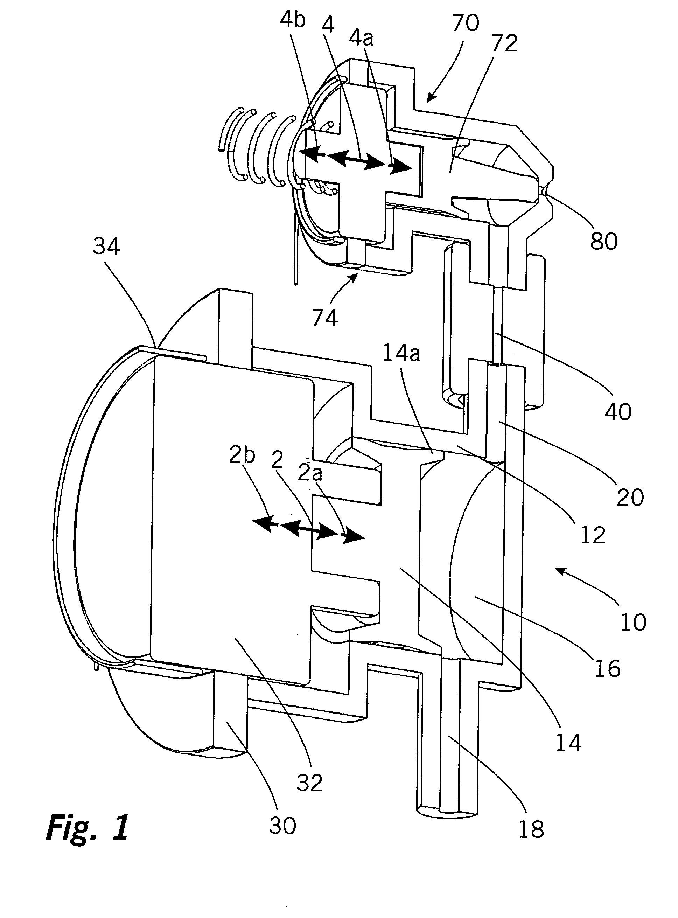

Fig. 1 zeigt eine erste Ausführungsform einer erfindungsgemäßen Austragvorrichtung. Diese weist eine Pumpvorrichtung 10 auf, die über einen Kanal 40 mit einem Auslassventil 70 verbunden ist. Nicht dargestellt sind in Fig. 1 weitere Komponenten der Austragvorrichtung, die weitgehend mit denen aus dem Stand der Technik bekannten Komponenten übereinstimmen. So ist ein die Austragvorrichtung umgebendes Gehäuse mit einem Mediumreservoir nicht dargestellt. Weiterhin verfügt die Austragvorrichtung über ein nicht dargestelltes Steuergerät, welches beispielsweise über Taster bedienbar ist und welches zur Steuerung eines Austragvorgangs vorgesehen ist.

Die Pumpvorrichtung 10 weist einen Pumpenzylinder 12 auf, der gemeinsam mit einem Kolben 14 einer Druckkammer 16 begrenzt. Der Kolben weist eine umlaufende Kolbenlippe 14a auf, die flüssigkeitsdicht am Pumpenzylinder anliegt. Durch die Beweglichkeit des Kolbens 14 ist das Volumen der Druckkammer veränderbar 16 und in der Druckkammer 16 befindliche Flüssigkeit mit einem Druck beaufschlagbar. In die Druckkammer 16 münden ein Einlasskanal 18 sowie ein Auslasskanal 20, der über den Verbindungskanal 40 mit dem Auslassventil 70 verbunden ist. Der Einlasskanal 18 und der Auslasskanal 20 sind bezogen auf eine Bewegungsrichtung 2 des Kolbens 14 versetzt angeordnet, so dass bei einer Bewegung des Kolbens in die Richtung 2a zunächst der Einlasskanal 18, der zu dem nicht dargestellten Mediumreservoir führt, von der Druckkammer getrennt wird.

Linksseitig schließt sich an dem Kolben 14 eine Spulenaktuatoreinrichtung auf, wobei diese einer Aktuatorspule 30 sowie einen durch die Aktuatorspule 30 umgebenen Aktuatorspulenkern 32 umfasst. Der Aktuatorspulenkern 32 ist durch eine Steckverbindung fest mit dem Kolben 14 verbunden, so dass der Aktuatorspulenkern sich stets gemeinsam mit dem Kolben 14 bewegt.

Darüber hinaus wird der Aktuatorspulenkern 32 von der Messspule 34 umgeben, die parallel zur Aktuatorspule 30 verläuft.

### Die Druckerzeugung in der Druckkammer 16 erfolgt folgendermaßen:

Durch das nicht dargestellte Steuergerät wird ein Strom in die Aktuatorspule 30 eingeleitet, der die Erzeugung eines Magnetfeldes im Bereich des Aktuatorspulenkerns 32 bewirkt. Die Stärke dieses Magnetfeldes ist von der Stromstärke in der Aktuatorspule 30 abhängig. Das Magnetfeld bewirkt, dass der Aktuatorspulenkern 32, der zumindest Abschnittsweise permanentmagnetisch ausgebildet ist, mit einer Kraft in Richtung 2a oder 2b beaufschlagt wird. Eine Kraftbeaufschlagung in Richtung 2a drückt auch den Kolben 14 in Richtung 2a auf die Druckkammer 16 zu. Sobald der Einlasskanal 18 von der Kolbenlippe 14a des Kolbens 14 überschritten wurde, führt diese Kraft zur Erzeugung eines Drucks in der volumenverringerten Druckkammer 16. Dieser Druck wirkt auch auf den Ventilkörper 72 des Auslassventils 70, welcher infolgedessen in Richtung 4b verlagert wird und so die zuvor vom Ventilkörper 72 verschlossene Auslassöffnung 80 freigibt. Durch dieses Öffnen der Auslassöffnung 80 wird somit der Austragvorgang bewirkt.

Der Austragvorgang endet, sobald die Kraftbeaufschlagung des Kolbens 14 entfällt. Dies kann durch ein Abschalten der Bestromung der Aktuatorspule 30 bewirkt werden. Alternativ ist es auch möglich, durch eine zusätzliche Spuleneinrichtung 74 am Auslassventil 70 den Ventilkörper 72 gegen den Flüssigkeitsdruck wieder in die Schließstellung der Fig. 1 zu drücken und somit den Austragvorgang zu beenden.

Nach Beendigung des Austragvorgangs wird die Aktuatorspule 30 mit einem umgepolten Strom bestromt, wodurch eine Kraftbeaufschlagung des Aktuatorspulenkerns 32 und des Kolbens 14 in Richtung 2b erfolgt. Da zu diesem Zeitpunkt das Auslassventil 70 wieder geschlossen ist, führt die resultierende Vergrößerung der Druckkammer 16 zu einem Unterdruck, der nach Überfahren des Einlasskanals 18 durch die Kolbenlippe 14 ein Ansaugen von Medium aus dem Mediumreservoir bewirkt.

Durch die beschriebene Ansteuerung der Aktuatorspule 30 ist eine Kraftbeaufschlagung des Kolbens 14 mit einer weitgehend definierten Kraft und damit eine Druckerzeugung mit weitgehend definiertem Druck möglich. Wenn an den in der Druckkammer 16 zu erzeugenden Druck besonders hohe Anforderungen hinsichtlich der Einhaltung eines Soll-Druckwertes gestellt werden, muss einbezogen werden, dass es zwischen dem Kolben 14 und der Zylinderwandung 12 zu Reibungskräften kommt, die bei der Druckbeaufschlagung entgegen der Bewegungsrichtung des Kolbens 14 wirken. Um diese Reibungskräfte bezüglich ihrer Höhe zu erfassen, wird vor Erstbefüllung der Druckkammer 16 eine Messung mit der Messspule 34 durchgeführt. Dabei wird der Kolben 14 durch eine definierte Stromstärke in der Aktuatorspule 30 aus seiner ersten Endlage in seine zweite Endlage verfahren und gleichzeitig mit der Messspule 34 erfasst, wie lange dieser Vorgang dauert. Je höher die Reibungskräfte sind, desto größer ist die Zeitspanne der Bewegung.

Diese erfasste Zeitspanne kann anschließend herangezogen werden, um durch eine Variation der Stromstärke in der Aktuatorspule 30 während der Druckbeaufschlagung des Mediums in der Druckkammer 16 die Reibungskräfte zu kompensieren und die gewünschten Druckverhältnisse in der Druckkammer 16 herzustellen.

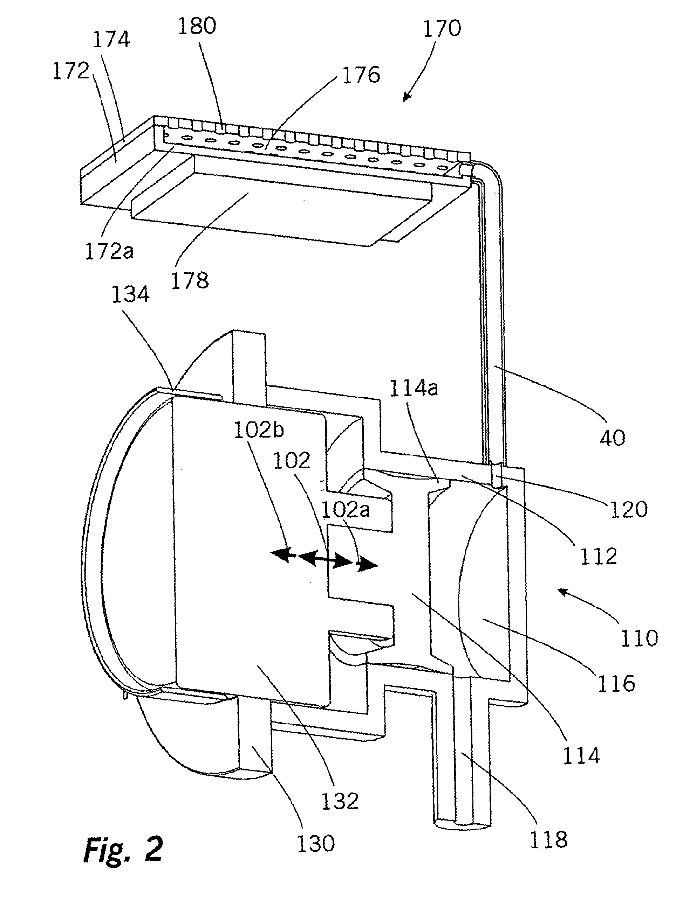

Die Austragvorrichtung gemäß der Fig. 2 entspricht weitgehend der Austragvorrichtung der Fig. 1. Hinsichtlich der Pumpvorrichtung 110 bestehen keinerlei Unterschiede, so dass das bereits zur Ausführungsform der Fig. 1 gesagte auch hierfür gilt. Abweichend von der Ausführungsform der Fig. 1 ist kein Auslassventil vorgesehen, sondern stattdessen ein über eine Leitung 140 mit der Druckkammer 116 verbundener Vernebler 170. Dieser Vernebler besteht aus einem Gehäuse 172, welches auf seiner Oberseite durch eine Lochplatte 174 abgeschlossen ist, wobei die Lochplatte 174 und das Gehäuse 172 gemeinsam einer Auslasskammer 176 einschließen. Auf der der Lochplatte 174 abgewandten Seite des Gehäuses 172 ist an einem Vibrationswandungsabschnitt 172a ein Vibrationspiezo 178 vorgesehen, der durch hochfrequentes Vibrieren eine Vibration des Vibrationswandungsabschnitts 172a erzeugen kann.

Diese Vibrationen in dem Vibrationswandungsabschnitts 172a führen zu einer hochfrequenten Volumenänderung der Auslasskammer 176 durch die das in der Kammer 176 eingeschlossene Medium durch Auslassöffnungen 180 der Lochplatte 174 durchgepresst wird und in Form eines Nebels entweicht.

Die Darstellung der Fig. 2 ist nicht maßstabsgerecht. Der Vernebler 170 ist im Verhältnis zur Pumpvorrichtung 110 deutlich vergrößert dargestellt. Das Volumen der Druckkammer 116 ist daher in der Realität um ein Vielfaches größer als das Volumen der Auslasskammer 176, so dass ein lang andauernder Austragvorgang durch das Medium in der Druckkammer 116 gespeist werden kann.

Im Betrieb wird die Austragvorrichtung der Fig. 2 derart von dem nicht dargestellten Steuergerät angesteuert, dass nur ein sehr geringer Überdruck in der Druckkammer 116 erzeugt wird. Dieser geringe Überdruck sorgt dafür, dass die Auslasskammer 176 permanent in einem befüllten Zustand verbleibt, ohne dass der durch die Pumpvorrichtung 110 erzeugte Druck zu einem von der Vibration der Vibrationswandung 172a unabhängigen Austritt des Mediums durch die Auslassöffnungen 180 führt.

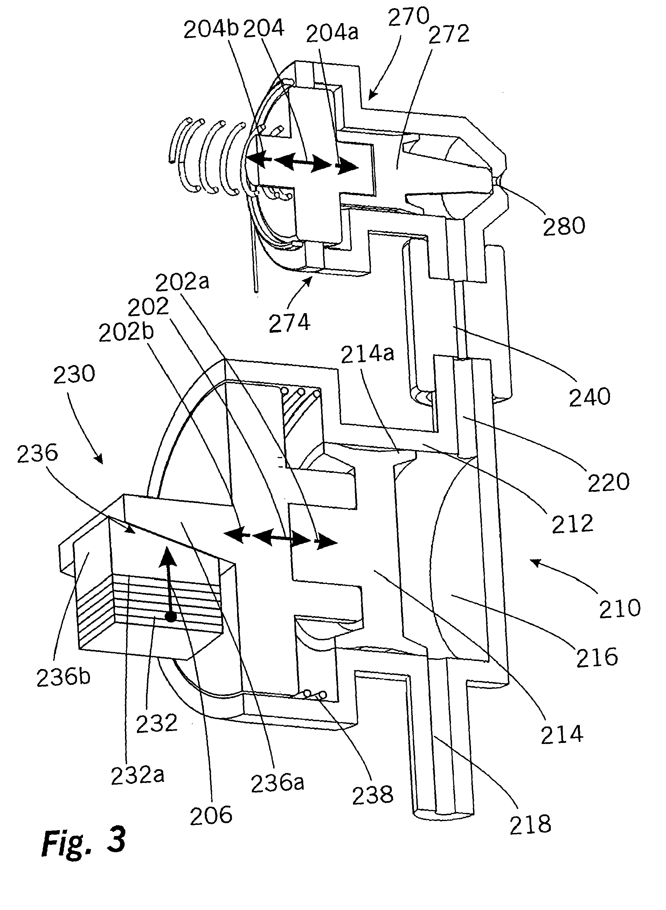

Bei der Ausführungsform der Fig. 3 entspricht der Aufbau der Ausführungsform der Fig. 1 mit der Ausnahme, dass der Kolben 214 nicht durch eine Spulenaktuatoreinrichtung kraftbeaufschlagbar ist, sondern durch eine Piezoaktuatoreinrichtung 230. Diese Piezoaktuatoreinrichtung 230 umfasst einen Piezo-Stack 232, der dafür ausgebildet ist, beim Anlegen einer Spannung seine Ausdehnung in Richtung 206 zu vergrößern. Diese Richtung 206 schließt mit der Bewegungsrichtung 202 des Kolbens 214 einen rechten Winkel ein. Zur Übertragung der Kraft vom Piezo-Stack 232 auf den Kolben 214 ist ein Umsetzer 236 vorgesehen, der aus zwei Keilelementen 236a, 236b besteht. Das Keilelement 236b ist am beweglichen Ende 232a des Piezo-Stacks 232 vorgesehen. Das andere Keilelement 236a ist auf der der Druckkammer 216 abgewandten Seite des Kolbens 214 auf diesen aufgesteckt. Die Keilflächen schließen mit der Bewegungsrichtung 202 einen Winkel von etwa 15° ein. Dies bewirkt, dass eine Verlagerung des beweglichen Endes 232a des Piezo-Stacks 232 zu einer erheblich größeren Verlagerung des Kolbens 214 führt. Eine vergleichsweise kleine Bewegung des Piezo-Stacks 232 kann somit einen deutlich größeren Kolbenhub bewirken.

Die dargestellte Gestaltung erlaubt es demnach, die durch den Piezo-Stack 232 erzeugte Kraft zur Druckerzeugung in der Druckkammer 216 zu verwenden. Da der Umsetzer 236 derart gestaltet ist, dass eine Kraftbeaufschlagung des Kolbens 214 lediglich in Richtung 202a möglich ist, ist zusätzlich eine Rückstellfeder 238 vorgesehen, die bei Wegfall der durch den Piezo-Stack 232 erzeugten Kraft den Kolben 214 zurück in seine Ausgangsstellung drückt.

## Patentansprüche

1. Austragvorrichtung für ein flüssiges pharmazeutisches Medium mit
- einem Reservoir zur Aufbewahrung des Mediums,
- einer Druckbeaufschlagungsvorrichtung (10; 110; 210) zur Förderung des Mediums und
- mindestens einer Auslassöffnung (80; 180; 280) zur Abgabe des Mediums an eine Umgebung,
wobei die Druckbeaufschlagungsvorrichtung (10; 110; 210) eine Druckkammer (16; 116; 216) aufweist, deren Inhalt mittels eines translativ beweglichen Kolbens (14; 114; 214) druckbeaufschlagbar ist,
**gekennzeichnet durch**
- eine Piezoaktuatoreinrichtung (232, 236) mit einem Piezoaktuator (232) , der mit dem Kolben (214) wirkgekoppelt ist oder
- eine Spulenaktuatoreinrichtung (30, 32; 130, 132) mit einer Aktuatorspule (30; 130) und einem Aktuatorspulenkern (32; 132), der **durch** Bestromung der Aktuatorspule (30; 130) gegenüber der Aktuatorspule (30; 130) kraftbeaufschlagbar ist,
wobei entweder die Aktuatorspule (30; 130) oder der Aktuatorspulenkern (32; 132) ortsfest zum Kolben ausgebildet ist.

2. Austragvorrichtung nach Anspruch 1,
**dadurch gekennzeichnet, dass**
die Piezoaktuatoreinrichtung (232, 236) über einen Umsetzer (236) mit dem Kolben (214) wirkgekoppelt ist, wobei der Umsetzer (236) dafür ausgebildet ist, den Kolben (214) in Folge einer Verformung des Piezoaktuators (232) um eine Weglänge L1 um eine Weglänge L2 zu verlagern, die größer als L1 ist.

3. Austragvorrichtung nach Anspruch 1,
**dadurch gekennzeichnet, dass**
- die Aktuatorspule (30; 130) ortsfest zu einem Pumpengehäuse (12; 112) vorgesehen ist und der Aktuatorspulenkern (32; 132) ortsfest zum Kolben (14; 114) angeordnet ist oder
- der Aktuatorspulenkern ortsfest zum Pumpengehäuse vorgesehen ist und die Aktuatorspule ortsfest am Kolben vorgesehen ist.

4. Austragvorrichtung nach einem der vorstehenden Ansprüche,
**dadurch gekennzeichnet, dass**
eine Messeinrichtung (32, 34; 132, 134) vorgesehen ist, mittels derer die Auslenkung des Kolbens (14; 114) zu einem Pumpengehäuse (12; 112) erfassbar ist.

5. Austragvorrichtung nach Anspruch 4,
**dadurch gekennzeichnet, dass**
die Messeinrichtung (32, 34; 132, 134) eine Messspule (34; 134) aufweist, die zur Erfassung der Lage eines Messspulenkerns (32; 132) relativ zur Messspule (34; 132) ausgebildet ist.

6. Austragvorrichtung nach einem der vorstehenden Ansprüche,
**gekennzeichnet durch**
eine mit der Druckkammer (116) verbundene Auslasskammer (176), wobei die Auslasskammer (176) **durch** eine Mehrzahl von Auslassöffnungen (180) mit der Umgebung verbunden ist und wobei die Auslasskammer (176) **durch** einen Wandungsabschnitt (172a) begrenzt wird, der **durch** einen Vibrationsaktuator (178) in einen Vibrationszustand versetzbar ist.

7. Austragvorrichtung nach einem der vorstehenden Ansprüche,
**gekennzeichnet durch**
ein Steuergerät, welches mittels der Piezoaktuatoreinrichtung (232, 236) oder der Spulenaktuatoreinrichtung (30, 32; 130, 132) eine Kraftbeaufschlagung des Kolbens (14; 114; 214) steuert, wobei vorzugsweise das Steuergerät zusätzlich dafür ausgebildet ist
- auf den Kolben (14; 114; 214) wirkende Reibungs- und/oder Federkräfte **durch** eine Auswertung der von der Messeinrichtung (32, 34; 132, 134) während einer Verlagerung des Kolbens (14; 114; 214) ermittelten Werte zu erfassen und/oder
- bei der Inbetriebnahme der Austragvorrichtung mittels einer Messung der Verlagerung des Kolbens (14; 114; 214) zu ermitteln, ob in einem Strömungspfad zwischen der Pumpvorrichtung (10; 110) und der Austragöffnung (80; 180) noch Luft eingeschlossen ist.
